# EUROPÄISCHE PATENTSCHRIFT

(11) **EP 2 334 350 B1**
(45) Veröffentlichungstag und Bekanntmachung des Hinweises auf die Patenterteilung: **20.04.2016**
(21) Anmeldenummer: 09778293.2
(22) Anmeldetag: 02.09.2009
(51) Int. Cl.: A61M 1/36, B01D 65/00

(54) **VORRICHTUNG ZUM BEFÜLLEN EINES FILTERS UND VERFAHREN HIERZU**
DEVICE FOR FILLING A FILTER, AND METHOD THEREFOR
DISPOSITIF POUR REMPLIR UN FILTRE ET PROCÉDÉ ASSOCIÉ

(30) Priorität: 02.09.2008 DE 102008045422
(43) Veröffentlichungstag der Anmeldung: 22.06.2011
(73) Patentinhaber: Fresenius Medical Care Deutschland GmbH, 61352 Bad Homburg (DE)
(72) Erfinder: BEDEN, Josef, 55252 Mainz-Kastel (DE); BADO, Itka, 61348 Bad Homburg (DE); VERCH, Georg, 65191 Wiesbaden (DE)
(74) Vertreter: Laufhütte, Dieter
(86) Internationale Anmeldenummer: PCT/EP2009/006372
(87) Internationale Veröffentlichungsnummer: WO 2010/025912

(56) Entgegenhaltungen:
- EP-A1- 0 723 463
- EP-A1- 0 747 074
- EP-A1- 1 100 559
- EP-A1- 1 395 311
- EP-A1- 1 457 218
- EP-A1- 1 707 226
- DE-C1- 10 011 208
- US-A1- 2007 185 430

## Beschreibung

Die vorliegende Erfindung betrifft eine Vorrichtung zum Befüllen eines medizinischen Filters und ein Verfahren hierzu.

Derartige medizinische Filter können beispielsweise Dialysatoren sein. Dialysatoren können dabei ein Bündel von Hohlfasermembranen aufweisen, die in einem Gehäuse eingefaßt sind und dabei eine Blutseite und eine Dialysatseite ausbilden, die durch die Vielzahl von Hohlfasermembranen voneinander getrennt sind. Ganz grundsätzlich, aber insbesondere bei der Dialyse, stellt sich vor der Inbetriebnahme eines medizinischen Filters, insbesondere Dialysators das Problem, diesen Filter vollständig zu entlüften bzw. zu befüllen. Für das Entlüften des Filters wird daher der Filter mit einer Flüssigkeit vorgespült und dadurch entlüftet. Bei Dialysatoren wird dies in der Regel während des Aufrüstvorganges der Dialysemaschine mit einer isotonischen Kochsalzlösung (NaCl-Lösung) durchgeführt, wobei die NaCl-Lösung mit einem Antikoagulanz wie z.B. Heparin versetzt sein kann.

Aus der DE 100 11 208 C1 ist eine Vorrichtung zum Befüllen eines Blutschlauchsatzes mit einem Dialysefilter bekannt, bei welcher der Blutschlauchsatz durch Flüssigkeit befüllt wird, welche durch die Membran von der Dialysatseite auf die Blutseite des Dialysefilters übertritt. Das Befüllen erfolgt dabei in mehreren Schritten unter Zirkulation von Flüssigkeit und Luft im Blutschlauchsatz, wobei in einem der Schritte über einen Füllstandssensor in der venösen Blasenkammer ermittelt wird, ob der Flüssigkeitspegel einen gewissen Wert erreicht hat und damit genügend Luft aus dem Blutschlauchset abgelassen wurde. Die Befüllung des Blutschlauchsatzes wird dann durch den Druckanstieg im Blutschlauchsatz überprüft.

Beim Vorbereiten bzw. Füllen des Filters und auch des extrakorporalen Blutkreislaufes wird gemäß einem anderen Vorgehen beispielsweise bei der "continuous veno-venous hemodialysis (CWHD)" oder "continuous veno-venous hemodiafiltration (CWHDF)" oftmals der Filter zusammen mit dem Schlauchsystem derart gefüllt, dass die einströmende Flüssigkeit bei einem senkrecht angeordneten Dialysator auf der Blutseite zunächst von unten einläuft. Somit wird die Luft ideal nach oben verdrängt und z.B. über den oberen venösen Anschluß des Dialysators in einen Abfallbeutel gefördert.

Da im Gegenstromverfahren das Dialysat mit entgegengesetzter Flußrichtung die Dialysatseite im Vergleich zur Blutseite durchströmt, sind Zu- und Ablauf dialysatseitig entgegengesetzt zu Zu- und Ablauf der Blutseite des Dialysators angeordnet. Beim Befüllen des Filters ergibt sich somit das Problem, dass bei einer Befüllung der Dialysatseite über die Membran von einer Blutseite aus, insbesondere bei senkrecht angeordnetem Dialysator, sich der Ablauf der Dialysatseite am unteren Ende benachbart zum Zulauf des Dialysators auf der Blutseite befindet, so dass sich bei einer Befüllung über die Membran nur eine teilweise Befüllung auf der Dialysatseite einstellt. Eine vollständige Entlüftung wird nur gewährleistet, wenn der Filter nach Befüllung der Blutseite und vor dem Füllen der Dialysatseite gedreht wird.

Dieses Problem wird bei vorbekannten System dadurch gelöst, dass dem Bedienungspersonal angezeigt wird, den Filter nach Befüllung der Blutseite entsprechend zu drehen. Ein weiterer bekannter Lösungsansatz besteht darin, den Blutkreislauf bei umgekehrter Konnektion durch Flußumkehr auf der Blutseite zu befüllen und hierdurch eine Drehung unnötig zu machen. Ein derartiger Ansatz ist jedoch sehr aufwendig, da zusätzliche Pumpen und Sensoreinrichtungen benötigt werden.

Ferner hat sich herausgestellt, dass ein Befüllen eines Filters mit einem Drehvorgang eine besonders zuverlässige Entlüftung bzw. Befüllung des Filters ermöglicht. Diese Vorgehensweise ist in der Klinik etabliert und anerkannt. Von daher wäre es wünschenswert, auch weiterhin zuerst die Blutseite von unten nach oben zu füllen, um hierdurch sicher die Luft aus der Blutseite zu verdrängen, und danach den Dialysator zu drehen, um anschließend die Dialysatseite zu entlüften und zu befüllen. Diese Vorgehensweise der Befüllung über die Membran von der Blutseite aus in die Dialysatseite hinein weist ferner den Vorteil auf, dabei auch die Poren der Membranen zu entlüften. Im Normalfall ist dabei während der späteren Behandlung der Bluteinlass am oberen Ende des Dialysators. Diese Anordnung ist bereits deshalb vorteilhaft, weil dadurch das Blut nicht gegen die Schwerkraft gefördert werden muss, was mit einer geringeren Belastung für das Blut einhergeht. Wird jedoch bei dieser Vorgehensweise die Drehung des Filters nach Aufforderung vergessen oder der Dialysator schon zu Beginn an falsch konnektiert, so kann im allgemeinen die Füllung der Dialysatseite nicht vollständig erfolgen und die Effektivität der Behandlung sinkt durch Reduzierung der effektiven Filterfläche. Ferner kann es insbesondere bei Systemen, die mittels Waagen eine Dialysat/Filtrat-Bilanzierung vornehmen, zu Alarmmeldung/-auslösungen durch Restlufteinträge in die Bilanzierungsbehältnisse kommen. Weitere Nachteile bestehen noch darin, dass durch verbliebene Restluft im Filter bei der späteren Behandlung die Gerinnung angeregt werden kann, was zu teilweiser Verstopfung der Membranen kommen führen könnte.

Es ist daher die Aufgabe der vorliegenden Erfindung, eine Vorrichtung und ein Verfahren der eingangs genannten Art in vorteilhafter Weise weiterzubilden, Insbesondere dahingehend, dass ein medizinischer Filter sicher und zuverlässig befüllt bzw. eine fehlerhafte Befüllung eines medizinischen Filters sicher erkannt werden kann.

Diese Aufgabe wird erfindungsgemäß durch eine Vorrichtung zum Befüllen eines medizinischen Filters mit den Merkmalen des Patentanspruchs 1 gelöst. Danach ist vorgesehen, dass eine Vorrichtung zum Befüllen eines medizinischen Filters wenigstens einen Filter mit wenigstens einem ersten und einem zweiten Raum aufweist, die durch eine oder mehrere Membranen semipermeabel getrennt sind, wobei die Räume jeweils wenigstens einen Zu- und Ablauf aufweisen, und wobei die Vorrichtung eine Detektionseinheit mit ersten und zweiten Mitteln aufweist, wobei mittels der ersten Mittel Flüssigkeitsaustritt aus wenigstens einem der Räume erfaßbar ist und mittels der zweiten Mittel anhand des erfaßten Flüssigkeitsaustritts der Zustand der Befüllung des Filters ermittelbar ist. Eine derartige Vorrichtung zum Befüllen eines medizinischen Filters kann dabei Fördermittel zum Befüllen des Filters mit Flüssigkeit aufweisen, beispielsweise eine Kreislaufpumpe, die vor dem ersten Raum des Filters angeordnet ist und eine Abzug- bzw. Filtratpumpe nach dem Ablauf des zweiten Raumes. Durch die Erfassung des Flüssigkeitsaustritts aus wenigstens einem der Räume ist es möglich, auf den Zustand der Befüllung des Filters zu schließen. So bedeutet beispielsweise bei einem Befüllungsvorgang, bei dem der erste Raum in einem ersten Schritt befüllt wird, ein Flüssigkeitsaustritt aus dem ersten Raum, dass der erste Raum vollständig gefüllt ist. Auf den vorstehend beschriebenen Befüllungsvorgang eines senkrecht für den Befüllungsvorgang angeordneten Dialysators angewandt bedeutet dies, dass der Filter nunmehr zu drehen wäre, um den Filter dialysatseitig zu entlüften.

Erfindungsgemäß wird mittels der Vorrichtung zur Befüllung zunächst der erste Raum befüllt und nach der Füllung des ersten Raumes der Filter um ca. 180° gedreht und der zweite Raum über die Membran bzw. Membranen durch Flüssigkeitsübertritt aus dem ersten Raum befüllt. Die Membranen können dabei Hohlfasermembranen eines Dialysators sein. Diese Vorgehensweise erlaubt es, die klinisch anerkannte Vorgehensweise zum Befüllen bzw. Entlüften eines Dialysators vorrichtungsseitig mittels der Detektionseinheit zu überwachen. Dabei wird eine erfolgte bzw. fehlende Filterdrehung erfasst. Eine überwachte, vollständige Befüllung des Filters wird hierdurch möglich. Von Vorteil ist es, wenn die Zu- und Abläufe des ersten und zweiten Raumes derart angeordnet sind, dass der wenigstens eine Filter im Gegenstrom betreibbar ist.

Es kann vorgesehen sein, dass ein Befüllungsvolumen für die Befüllung des Filters vorgegeben und/oder in der Vorrichtung hinterlegt ist und dass mittels der zweiten Mittel der Detektionseinheit eine korrekte Befüllung des Filters ermittelt wird, wenn mittels der ersten Mittel nach Abgabe des Befüllungsvolumens an den Filter ein Flüssigkeitsaustritt aus dem zweiten Raum erfasst wird. Dieses Befüllungsvolumen kann sich dabei am Füllvolumen des Dialysators orientieren und ist vorteilhafterweise geringfügig größer bemessen, als das Füllvolumen eines Filters bzw. Dialysators. In das Befüllungsvolumen ist ebenfalls vorteilhafterweise das Totvolumen des zu verwendenden Schlauchsets mit einzubeziehen. Dadurch wird es möglich, beispielsweise durch Abgleich eines eingespeicherten Wertes mit dem mittels einer Fördereinrichtung erfassten geförderten Volumens vorrichtungsseitig zu erkennen, wenn das komplette Befüllungsvolumen für die Befüllung des Filters an den Filter abgegeben wurde. Insbesondere in dem Fall, in dem zunächst der erste Raum von unten nach oben befüllt, sodann der Filter um 180° gedreht und danach der zweite Raum über die Membran aus dem ersten Raum befüllt wird, befindet sich der Ablauf des zweiten Raumes am oberen Ende des Dialysators, so dass ein Flüssigkeitsaustritt erst dann erfolgen kann, wenn sowohl der erste und der zweite Raum des Filters und damit der gesamte Filter vollständig befüllt sind.

Es ist ferner denkbar, dass ein Befüllungsvolumen für die Befüllung des Filters vorgegeben und/oder in der Vorrichtung hinterlegt ist und dass mittels der zweiten Mittel der Detektionseinheit eine fehlerhafte Befüllung des Filters ermittelt wird, wenn mittels der ersten Mittel während der Abgabe des Befüllungsvolumens an den Filter ein Flüssigkeitsaustritt aus dem zweiten Raum erfaßt wird. In diesem Zusammenhang ist denkbar, dass bei einer senkrechten Anordnung mit einem unten angeordneten Zulauf eines ersten Raumes bei einer Befüllung des ersten Raumes zunächst der erste Raum von unten nach oben befüllt wird. Sollte der Filter anschließend nicht oder nicht vollständig gedreht werden, wird bei einer Befüllung der Dialysatseite über die Membran Flüssigkeit durch den nicht korrekt, d. h. dann weiterhin unten liegenden Ablauf des zweiten Raumes Flüssigkeit nach Außen abgegeben, so dass anhand dieses Flüssigkeitsaustritts aus dem zweiten Raum auf eine fehlerhafte Anordnung des Filters und damit auf eine fehlerhafte Befüllung des Filters geschlossen werden kann. Eine maschinen- bzw. vorrichtungsseitige Detektion der fehlerhaften Befüllung wird dadurch ermöglicht.

Es ist denkbar, dass die ersten Mittel wenigstens einen Sensor umfassen, der anhand von Gewicht und/oder Volumen und/oder Durchfluß und/oder Leitfähigkeit den Flüssigkeitsaustritt aus wenigstens einem der Räume erfaßt. So kann ein Sensor derart ausgebildet sein, dass er das Gewicht des aus dem beispielsweise zweiten Raum austretenden Fluids erfaßt. Genauso gut ist es jedoch zusätzlich oder als einzelne Meßmethode möglich, das Volumen des austretenden Fluids zu bestimmen. Darüber hinaus sind bereits jetzt in beispielsweise Dialysemaschinendurchfluß- und/oder Leitfähigkeitssensoren vorgesehen, mittels derer ebenfalls ein Flüssigkeitsaustritt detektiert werden kann. Sämtliche vorgenannten Sensoren werden bereits jetzt in entsprechenden Dialysemaschinen verwendet, so dass vorteilhafterweise bereits vorhandene Signalgeber zur Erfassung des Befüllungszustandes herangezogen werden können.

Es kann vorgesehen sein, dass nach dem zweiten Raum eine Auffangvorrichtung zur Aufnahme von aus dem zweiten Raum ablaufender Flüssigkeit vorgesehen ist und dass die ersten Mittel eine Wägeeinrichtung umfassen oder als Wägeeinrichtung ausgebildet sind, die das Gewicht der Auffangvorrichtung ermittelt. Eine derartige Wägeeinrichtung ist insbesondere bei CWHD- oder CVVHDF-Maschinen mit einer Befüllungseinrichtung eines Dialysators vorhanden, so dass für die erfindungsgemäße Vorrichtung auf bereits vorhandene Komponenten zugegriffen werden kann. So ist in diesem Zusammenhang denkbar, dass nach der Füllung der Blutseite durch Drehung des Filters und Durchlauf der beispielsweise entsprechend angeordneten Blut- und Filtratpumpe die Dialysatseite über die Membran gefüllt wird. Die Luft wird aus dem Filter nun in den Filtratbeutel gefördert. Sofern der Filter korrekt gedreht wurde, vergeht eine gewisse Zeit wegen der Einförderung des notwendigen Volumens, bis die Luft vollständig aus dem Filter verdrängt ist und am oberen Auslass des zweiten Raumes Flüssigkeit austritt. Diese Flüssigkeit gelangt dann in den Abfallbeutel und damit zur Waage. Erst nach Förderung dieses Volumens, das im wesentlichen dem Füllvolumen des Dialysators auf der Dialysatseite entspricht, wird eine Gewichtszunahme an der Waage detektiert. Somit wird es möglich, dieses verzögerte Gewichtssignal an der Waage zur Erkennung der erfolgten Filterdrehung und der vollständigen Befüllung des Filters zu verwenden. In dem Fall, in dem der Filter nicht korrekt gedreht oder schon zu Beginn falsch konnektiert war, fließt das Dialysat nach Befüllung des ersten Raumes ohne wesentliche Verzögerung direkt zum unteren Dialysatanschluß des Filters, das heißt dem Ablauf des zweiten Raumes, so dass die Zeitverzögerung bis zur Gewichtszunahme im Filtratbeutel ausbleibt, wodurch vorrichtungsseitig erkannt wird, dass eine fehlerhafte und unvollständige Befüllung des Filters vorliegt.

Es kann vorgesehen sein, dass die zweiten Mittel derart ausgebildet sind, dass in den zweiten Mitteln ein Schwellwert für den Flüssigkeitsaustritt hinterlegt ist und ein durch die ersten Mittel erfaßter, unterhalb eines vorgegeben Schwellwertes liegender Flüssigkeitsaustritt, der während der Befüllung des Filters auftritt, ausgeblendet wird und für die Beurteilung des Befüllungszustandes unberücksichtigt bleibt. Dadurch wird es möglich, Tolleranzen im Füllvolumen, insbesondere des Disposable-Schlauchsets zu berücksichtigen und die Sensitivität der Detektionseinheit einzustellen aber auch nachjustieren zu können.

Darüber hinaus ist denkbar, dass ein Teilbefüllungsvolumen für die Befüllung des Filters vorgegeben ist, wobei das Teilbefüllungsvolumen geringfügig größer ist als das Füllvolumen des ersten Raumes, und mittels der zweiten Mittel der Detektionseinheit der Zustand des Ablaufs aus dem zweiten Raum ermittelbar ist, wobei ein geöffneter Ablauf durch die zweiten Mittel erkannt wird, wenn nach Abgabe des Teilbefüllungsvolumens ein Flüssigkeitsaustritt aus dem zweiten Raum durch die ersten Mittel ermittelt wird und/oder ein geschlossener Ablauf des zweiten Raumes dadurch erkannt wird, wenn nach Abgabe des Teilbefüllungsvolumens kein Flüssigkeitsaustritt aus dem zweiten Raum durch die ersten Mittel ermittelt wird. Dadurch wird es möglich, sich den Umstand zu Nutze zu machen, wonach beim korrekten Füllen der Blutseite, d. h. des vollständigen Füllens des ersten Raumes bzw. beim Vorfüllen des Dialysatorzulaufs, geringfügig über das Füllvolumen des ersten Raumes befüllt wird, und die geringe Flüssigkeitsmenge, die vom ersten in den zweiten Raum übertritt und direkt über den sich unten liegenden Ablauf des zweiten Raumes in den Filtratbeutel fließt, für eine Detektion benutzt werden, ob eventuell ein Verschluß in der Zuleitung zum Abfallbeutel, beispielsweise eine vergessene Klemme vorliegt. Sollte eine Klemme und damit ein Verschluß vorhanden sein, kann keine Gewichtszunahme im Abfallbeutel detektiert werden. In diesem Zusammenhang ist denkbar, das hierfür zusätzlich genutzte Flüssigkeitsvolumen zu entfernen oder in die Befüllungsbilanzierung mit einzubeziehen, beispielsweise durch eine Anpassung des Schwellwertes. Insbesondere ist es in diesem Zusammenhang von Vorteil, dass relativ frühzeitig eine Erkennung eines Verschlusses der Abfallleitung möglich ist, so dass eventuell zusätzliche hieraus resultierende Komplikationen im Vorfeld vermieden werden können.

Es wird bevorzugt, wenn die Vorrichtung Teil eines medizinischen Gerätes, insbesondere einer Dialysemaschine ist. Besonders vorteilhaft ist es, wenn es sich dabei um eine CWHD- oder eine CVVHDF-Maschine handelt.

Die Erfindung betrifft weiter ein Verfahren zum Befüllen eines medizinischen Filters mit den Merkmalen des Anspruchs 10. Danach ist vorgesehen, dass beim Befüllen eines medizinischen Filters, wobei der Filter wenigstens einen ersten und einen zweiten Raum aufweist, die durch eine oder mehrere Membranen semipermeabel getrennt sind, und die Räume jeweils einen Zu- und Ablauf aufweisen und nacheinander befüllt werden, der Flüssigkeitsaustritt aus wenigstens einem der Räume erfaßt und anhand des erfaßten Flüssigkeitsaustritt der Zustand der Befüllung des Filters ermittelt wird.

Erfindungsgemäß wird zur Befüllung zunächst der erste Raum befüllt und nach Füllung des ersten Raumes der Filter um ca. 180° gedreht und der zweite Raum über die Membran bzw. Membranen durch Flüssigkeitsübertritt aus dem ersten Raum befüllt. Dabei wird eine erfolgte bzw. fehlende Filterdrehung erkannt.

Darüber hinaus ist denkbar, dass eine korrekte Befüllung des Filters ermittelt wird, wenn mittels der ersten Mittel nach Abgabe eines vorgegebenen Befüllungsvolumens an den Filter ein Flüssigkeitsaustritt aus dem zweiten Raum erfaßt wird und/oder eine fehlerhafte Befüllung des Filters ermittelt wird, wenn mittels der ersten Mittel während der Abgabe eines vorgegebenen Befüllungsvolumens an den Filter ein Flüssigkeitsaustritt aus dem zweiten Raum erfaßt wird. Das Befüllungsvolumen kann sich dabei an dem Füllvolumen des Filters sowie des Totvolumens des Schlauchsets orientieren und beispielsweise in einer Dialysemaschine oder einer Vorrichtung zum Befüllen eines medizinischen Filters eingespeichert sein. Genauso gut oder zusätzlich ist möglich, mittels eines speziell dafür vorgesehenen Befüllungsbeutels mit Befüllungsflüssigkeit das für die Befüllung des Filters notwendige Flüssigkeitsvolumen vorzuportionieren und für die Befüllung diesen Befüllungsbeutel an das Schlauchset anzuschließen.

Außerdem ist denkbar, dass der Flüssigkeitsaustritt aus wenigstens einem der Räume anhand des Gewichts und/oder Volumens und/oder Durchflusses und/oder Leitfähigkeit der austretenden Flüssigkeit aus dem Filter erfaßt wird.

Ferner ist möglich, dass nach dem zweiten Raum eine Auffangvorrichtung zur Aufnahme von aus dem zweiten Raum ablaufender Flüssigkeit vorgesehen ist und dass zur Ermittlung des Flüssigkeitsaustritts das Gewicht der Auffangvorrichtung erfaßt und ausgewertet wird.

Von Vorteil ist es, wenn ein Schwellwert für den Flüssigkeitsaustritt vorgegeben wird und dass ein unterhalb eines vorgegeben Schwellwertes vorliegender Flüssigkeitsaustritt, der während der Befüllung des Filters auftritt, ausgeblendet wird und für die Beurteilung des Befüllungszustandes unberücksichtigt bleibt. In diesem Zusammenhang ist es denkbar, einen Schwellwert als prozentualen Wert des Befüllungsvolumens, also des Volumens, das für die Befüllung des Filters und des zugehörigen Schlauchsets notwendig ist, anzusetzen.

Der Zustand des Ablaufs aus dem zweiten Raum kann dadurch ermittelt werden, dass ein Teilbefüllungsvolumen für die Befüllung des Filters vorgegeben wird, wobei das Teilbefüllungsvolumen geringfügig größer ist als das Füllvolumen des ersten Raumes, und wobei nach Befüllen des ersten Raumes und vor einem Drehen des Filters ein geöffneter Ablauf des zweiten Raumes dadurch erkannt wird, dass nach Abgabe des Teilbefüllungsvolumens ein Flüssigkeitsaustritt aus dem zweiten Raum ermittelt wird und/oder ein geschlossener Ablauf des zweiten Raumes dadurch erkannt wird, dass nach Abgabe des Teilbefüllungsvolumens kein Flüssigkeitsaustritt aus dem zweiten Raum ermittelt wird.

Weitere Einzelheiten und Vorteile der Erfindung sollen nun anhand eines in der Zeichnung dargestellten Ausführungsbeispiels näher erläutert werden. Es zeigen:
- Figur 1:: eine schematische Darstellung des Befüllungsvorgangs der Blutseite eines Dialysators,
- Figur 2:: eine schematische Darstellung des Befüllungsvorgangs der Dialysatseite eines Dialysators bei korrekter Drehung,
- Figur 3:: eine schematische Darstellung der Befüllung der Dialysatseite eines Dialysators bei fehlerhaftem Ablauf bzw. unvollständiger Füllung,
- Figur 4:: eine schematische Darstellung des Befüllungsvorgangs eines Dialysators ohne Filter-Drehung,
- Figur 5:: eine schematische Darstellung der Erkennung eines offenen Anschlusses bzw. Ablaufs auf der Dialysatseite und
- Figur 6:: eine schematische Darstellung der Erkennung eines verschlossenen Ablaufs auf der Dialysatseite.

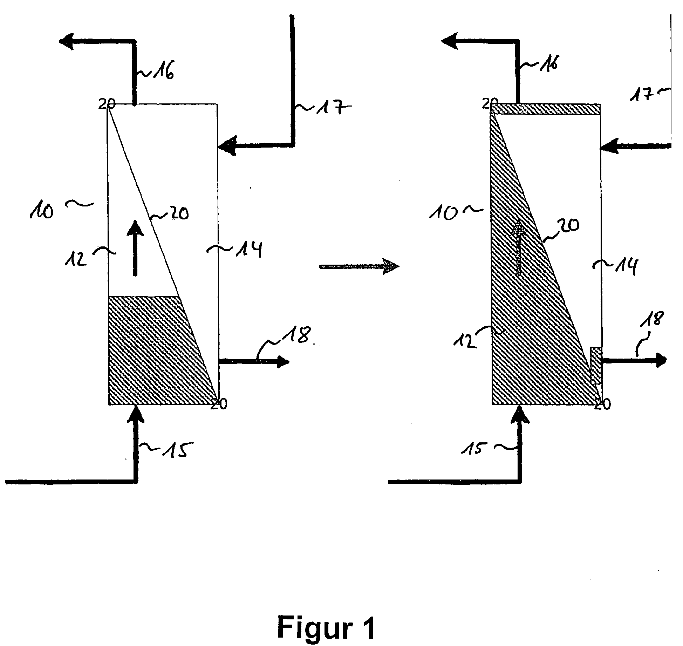

Figur 1 zeigt schematisch die Befüllung eines medizinischen Filters 10, hier eines Dialysators 10, bei dem der arterielle Zulauf 15, also der Zulauf der Blutseite 12 am unteren Ende des senkrecht angeordneten Dialysators 10 angeordnet ist.

Die erfindungsgemäße Vorrichtung ist dabei Teil einer Dialysemaschine, die vorzugsweise als Dialysatoren 10 ausgebildete medizinische Filter 10 mit mehreren Hohlfasermembranen 20 verwendet, wobei die bündelartig in einem Dialysatorgehäuse zusammengefaßten Hohlfasermembranen 20 die Blutseite 12 semipermeabel von der Dialysatseite 14 trennen. Die Blutseite 12 und die Dialysatseite 14 des Dialysators 10 weisen dabei jeweils gesonderte Zu- und Abläufe 15, 16, 17, 18 auf. Die Blutseite 12 weist dabei für den Befüllungsvorgang einen unten liegenden arteriellen Zugang 15 und einen oben liegenden venösen Rücklauf 16 auf, während die Dialysatseite einen oben liegenden Dialysatzulauf 17 und einen unten liegenden Filtratablauf 18 aufweist. Bevorzugt wird es, wenn die Detektionseinheit 30 der Vorrichtung zum Befüllen eines medizinischen Filters 10 durch bereits vorhandene Bestandteile der Dialysemaschine gebildet wird. Dabei ist es denkbar, dass die ersten Mittel, die den Flüssigkeitsaustritt aus beispielsweise der Dialysatseite 14 oder der Blutseite 12 des Dialysators 10 erfassen, durch Sensoren der Dialysemaschine gebildet werden. Die zweiten Mittel, anhand derer der Zustand der Befüllung des Filters 10 ermittelt wird, kann besonders vorteilhaft durch die Maschinensteuerung der Dialysemaschine gebildet werden oder auch durch ein gesondertes Steuerungsmodul, das mit der Maschinensteuerung der Dialysemaschine in Verbindung steht.

Wie in Figur 1 dargestellt, sind die Zu- und Abläufe des ersten und zweiten Raumes, also der Dialysatseite 14 und der Blutseite 12 des Dialysators 10 derart angeordnet, dass der Dialysator 10 im Gegenstrom betrieben wird. Für die Füllung des Filters 10 wird dabei zunächst die Blutseite 12 befüllt, wie dies in Figur 1 dargestellt ist. Dabei wird mittels nicht näher dargestellter Pumpen, beispielsweise der Kreislaufpumpe der Blutseite bzw. Blutpumpe des extrakorporalen Kreislaufs der Dialysemaschine eine Primerlösung, beispielsweise bestehend aus einer Natriumchloridlösung, die vorteilhafterweise mit Heparin versetzt ist, von unten nach oben befüllt. Dieser Befüllungsvorgang der Blutseite 12 des Filters 10 wird solange durchgeführt, bis wie in Figur 1 rechts dargestellt, die Blutseite 12 vollständig befüllt ist. Sodann erfolgt eine Drehung des Dialysators 10 um 180°.

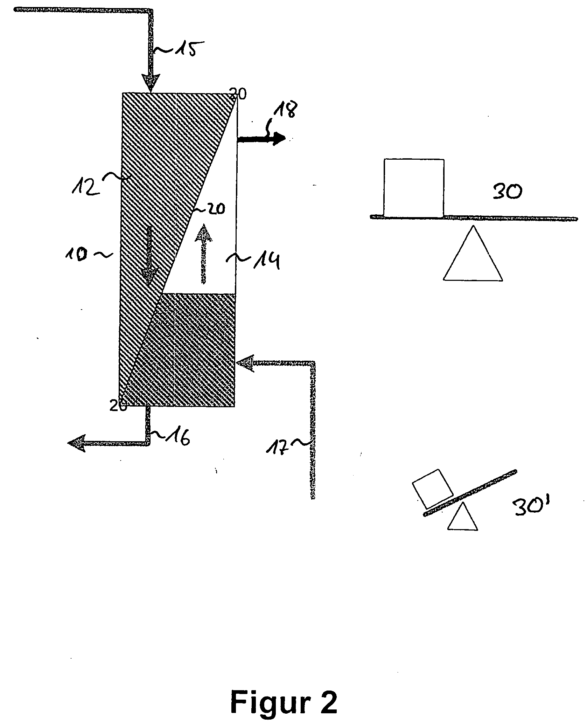

Nach der Drehung um 180° gegen den Uhrzeigersinn, wie dies in Figur 2 dargestellt ist, erfolgt eine Befüllung der Dialysatseite 14 über die Membranen des Dialysators 10. Dabei kann es vorgesehen sein, dass der venöse Rücklauf 16 und der Dialysatzulauf 17 für die Befüllung der Dialysatseite 14 abgeklemmt werden bzw. mittels einer nicht dargestellten Filtratpumpe am Filtratablauf 18 Flüssigkeit entzogen wird. Da sich der Filtratablauf 18 der Dialysatseite 14 am oberen Ende des Dialysators 10 befindet, kann es zu einem Flüssigkeitsaustritt aus dem Filtratablauf erst dann kommen, wenn die Dialysatseite 14 vollständig befüllt ist. Erst danach kann mittels einer Wiegeeinrichtung 30, die schematisch dargestellt ist, das durch den Flüssigkeitsaustritt aus dem Filtratablauf 18 erzeugte Gewichtssignal erfaßt werden. Durch die Maschinensteuerung wird das erfaßte Gewichtssignal in Relation mit dem hinterlegten Befüllungsvolumen des Dialysators 10 in Relation gesetzt, so dass eine vollständige Befüllung des Dialysators 10 erkannt wird. Dieses Gewichtssignal wird durch die Waage 30' erfasst, wie dies im unteren rechten Teil der Figur 2 dargestellt ist. Das Befüllungsvolumen berechnet sich dabei aus dem für die vollständige Füllung des Dialysators 10 notwendigen Volumens zuzüglich des Totvolumens für die Schlauchsysteme.

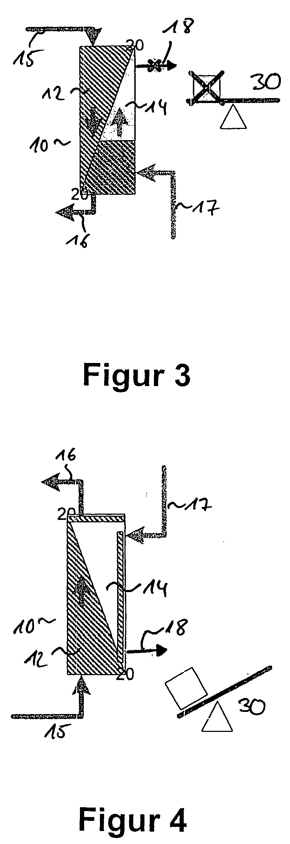

Figur 3 zeigt schematisch den Fall, bei dem mittels der Waage 30 nach dem abgeben des maximalen Befüllungsvolumens seitens der Befüllungsvorrichtung kein Waagensignal erfaßt wird. Dies deutet daraufhin, dass es zu Fehlern im Ablauf, gegebenenfalls Undichtigkeiten im Schlauchsystem und zu einer unvollständigen Füllung des Dialysators 10 gekommen ist.

Figur 4 zeigt den Fall, bei dem der Dialysator 10 nach vollständiger Befüllung der Blutseite 12 nicht um 180° gedreht wurde. Eine weitere Befüllung über die Membran- auf die Dialysatseite 14 führt unmittelbar zu einem Flüssigkeitsaustritt aus dem Filtratablauf 18, der sich bei der in Figur 4 gezeigten Anordnung im unteren Teil des Dialysators 10 befindet. Es kommt zu einem Flüssigkeitsaustritt, der mittels der Waage 30 unmittelbar erfaßt wird. In Relation gesehen mit dem Befüllungsvolumen wird maschinenseitig detektiert, dass es zu einem Waagensignal während des Befüllungsvorgangs gekommen ist, was maschinenseitig mit einer fehlenden Filterdrehung gleichgesetzt wird. In diesem Zusammenhang ist es denkbar, diese Fehlbedienung mittels einer Warnmeldung beispielsweise optisch und akustisch an der Dialysemaschine auszugeben.

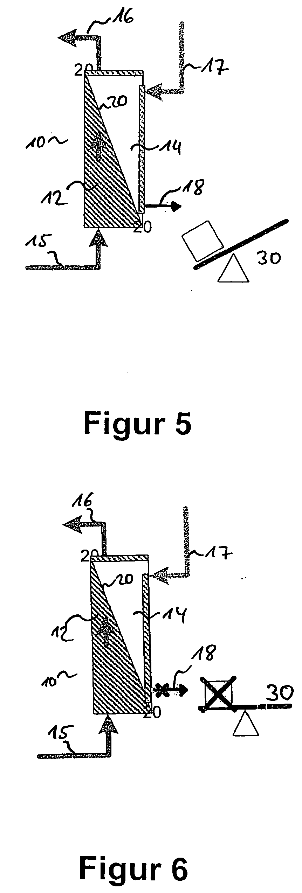

Figur 5 zeigt eine besondere Ausgestaltung der erfindungsgemäßen Vorrichtung und des erfindungsgemäßen Verfahrens. Dabei wird ein Teilbefüllungsvolumen für die Befüllung des Filters 10 vorgegeben, welches geringfügig größer ist als das Füllvolumen der Blutseite 12 bzw. das Volumen zur Vorfüllung der Dialysatoreingangsseite 17 liegt geringfügig über der min. nötigen Menge. Dieses Teilbefüllungsvolumen wird vollständig in den Dialysator 10 eingefördert, so dass es zu einem teilweisen Übertritt über die Membran 20 in die Dialysatseite 14 bzw. einem teilweisen Füllen der Dialysatseite 14 kommt. Dieser teilweise Flüssigkeitsübertritt kann mittels der Waage 30 detektiert werden. Maschinenseitig wird sodann anhand des Waagensignals in Verbindung mit der Information, dass das Teilbefüllungsvolumen vollständig abgegeben wurde, erkannt, dass der Filtratablauf 18 geöffnet ist.

Figur 6 zeigt dementsprechend den Fall, bei dem ebenfalls wie in Figur 5 das Teilbefüllungsvolumen in die Blutseite 12 des Dialysators 10 eingefördert wurde, jedoch der Filtratablauf 18 der Dialysatseite 14 versperrt ist. In diesem Fall ist es nicht möglich, mittels der Wägeeinrichtung 30 ein Waagensignal zu erhalten. Maschinenseitig wird aus dieser Signalkombination bestehend aus fehlendem Waagensignal in Verbindung mit vollständig eingeförderten Teilbefüllungsvolumen in die Blutseite 10 erkannt, dass der Filtratablauf 18 der Dialysatseite 14 des Dialysators 10 versperrt ist. Dieser fehlerhafte Zustand kann sodann maschinenseitig ebenfalls als optische und/oder akustische Warnmeldung beispielsweise über den Monitor der Dialysemaschine ausgegeben werden.

## Patentansprüche

1. Vorrichtung zum Befüllen eines medizinischen Filters, mit
wenigstens einem Filter (10) mit wenigstens einem ersten (12) und einem zweiten (14) Raum, die durch eine oder mehrere Membranen semipermeabel getrennt sind, wobei die Räume (12, 14) jeweils wenigstens einen Zu- und Ablauf (15, 17, 16, 18) aufweisen,
wobei mittels der Vorrichtung zunächst der erste Raum (12) befüllbar ist und nach Füllung des ersten Raumes der Filter (10) um ca. 180° drehbar ist, so dass sich der Ablauf des zweiten Raumes am oberen Ende des Dialysators befindet, und dann der zweite Raum (14) über die Membran bzw. Membranen durch Flüssigkeitsübertritt aus dem ersten Raum befüllbar ist, und
einer Detektionseinheit (30) mit ersten und zweiten Mitteln, wobei mittels der ersten Mittel Flüssigkeitsaustritt aus wenigstens dem zweiten Raum (14) erfassbar ist und mittels der zweiten Mittel anhand des erfaßten Flüssigkeitsaustritts der Zustand der Befüllung des Filters (10) ermittelbar ist, wobei eine erfolgte bzw. fehlende Filterdrehung erfassbar ist.

2. Vorrichtung zum Befüllen eines medizinischen Filters nach Anspruch 1, **dadurch gekennzeichnet, dass** die Zu- und Abläufe (15, 17, 16, 18) des ersten und zweiten Raumes derart angeordnet sind, dass der wenigstens eine Filter (10) im Gegenstrom betreibbar ist.

3. Vorrichtung zum Befüllen eines medizinischen Filters nach Anspruch 1 oder 2, **dadurch gekennzeichnet, dass** ein Befüllungsvolumen für die Befüllung des Filters (10) vorgegeben und/oder in der Vorrichtung hinterlegt ist und dass mittels der zweiten Mittel der Detektionseinheit (30) eine korrekte Befüllung des Filters ermittelt wird, wenn mittels der ersten Mittel nach Abgabe des Befüllungsvolumens an den Filter ein Flüssigkeitsaustritt aus dem zweiten Raum erfasst wird.

4. Vorrichtung zum Befüllen eines medizinischen Filters nach einem der vorhergehenden Ansprüche, **dadurch gekennzeichnet, dass** ein Befüllungsvolumen für die Befüllung des Filters (10) vorgegeben ist und/oder in der Vorrichtung hinterlegt ist und dass mittels der zweiten Mittel der Detektionseinheit (30) eine fehlerhafte Befüllung des Filters ermittelt wird, wenn mittels der ersten Mittel während der Abgabe des Befüllungsvolumens an den Filter ein Flüssigkeitsaustritt aus dem zweiten Raum erfasst wird.

5. Vorrichtung zum Befüllen eines medizinischen Filters nach einem der vorhergehenden Ansprüche, **dadurch gekennzeichnet, dass** die ersten Mittel wenigstens einen Sensor umfassen, der anhand von Gewicht und/oder Volumen und/oder Durchfluss und/oder Leitfähigkeit den Flüssigkeitsaustritt aus wenigstens einem der Räume erfasst.

6. Vorrichtung zum Befüllen eines medizinischen Filters nach einem der vorhergehenden Ansprüche, **dadurch gekennzeichnet, dass** nach dem zweiten Raum eine Auffangvorrichtung zur Aufnahme von aus dem zweiten Raum ablaufender Flüssigkeit vorgesehen ist und dass die ersten Mittel eine Wägeeinrichtung (30) umfassen oder als Wägeeinrichtung ausgebildet sind, die das Gewicht der Auffangvorrichtung ermittelt.

7. Vorrichtung zum Befüllen eines medizinischen Filters nach einem der vorhergehenden Ansprüche, **dadurch gekennzeichnet, dass** die zweiten Mittel derart ausgebildet sind, dass in den zweiten Mitteln ein Schwellwert für den Flüssigkeitsaustritt hinterlegt ist und ein durch die ersten Mittel erfasster, unterhalb eines vorgegebenen Schwellwertes liegender Flüssigkeitsaustritt, der während der Befüllung des Filters (10) auftritt, ausgeblendet wird und für die Beurteilung des Befüllungszustandes unberücksichtigt bleibt.

8. Vorrichtung zum Befüllen eines medizinischen Filters nach einem der vorhergehenden Ansprüche, **dadurch gekennzeichnet, dass** ein Teilbefüllungsvolumen für die Befüllung des Filters (10) vorgegeben ist, wobei das Teilbefüllungsvolumen geringfügig größer ist als das Füllvolumen des ersten Raumes (12), und mittels der zweiten Mittel der Detektionseinheit der Zustand des Ablaufs aus dem zweiten Raum ermittelbar ist, wobei ein geöffneter Ablauf durch die zweiten Mittel erkannt wird, wenn nach Abgabe des Teilbefüllungsvolumens ein Flüssigkeitsaustritt aus dem zweiten Raum durch die ersten Mittel ermittelt wird und/oder ein geschlossener Ablauf des zweiten Raumes dadurch erkannt wird, wenn nach Abgabe des Teilbefüllungsvolumens kein Flüssigkeitsaustritt aus dem zweiten Raum durch die ersten Mittel ermittelt wird.

9. Vorrichtung zum Befüllen eines medizinischen Filters nach einem der vorhergehenden Ansprüche, **dadurch gekennzeichnet, dass** die Vorrichtung Teil eines medizinischen Gerätes, insbesondere einer Dialysemaschine ist.

10. Verfahren zum Befüllen eines medizinischen Filters (10),
wobei der Filter (10) wenigstens einen ersten (12) und einen zweiten (14) Raum aufweist, die durch eine oder mehrere Membranen (20) semipermeabel getrennt sind, und die Räume jeweils wenigstens einen Zu- und Ablauf (15, 17, 16, 18) aufweisen und nacheinander befüllt werden,
wobei zur Befüllung zunächst der erste Raum (12) befüllt wird und nach Füllung des ersten Raumes der Filter (10) um ca. 180° gedreht wird, so dass sich der Ablauf des zweiten Raumes am oberen Ende des Dialysators befindet,
und dann der zweite Raum (14) über die Membran bzw. Membranen durch Flüssigkeitsübertritt aus dem ersten Raum befüllt wird, und
wobei der Flüssigkeitsaustritt aus wenigstens dem zweiten Raum (14) erfasst und anhand des erfaßten Flüssigkeitsaustritts der Zustand der Befüllung des Filters ermittelt wird, wobei eine erfolgte bzw. fehlende Filterdrehung erkannt wird.

11. Verfahren zum Befüllen eines medizinischen Filters nach Anspruch 10, **dadurch gekennzeichnet, dass** die Zu- und Abläufe (15, 17, 16, 18) des ersten und zweiten Raumes (12, 14) derart angeordnet sind, dass der wenigstens eine Filter im Gegenstrom betrieben ist.

12. Verfahren zum Befüllen eines medizinischen Filters nach Anspruch 10 oder 11, **dadurch gekennzeichnet, dass** eine korrekte Befüllung des Filters (10) ermittelt wird, wenn mittels der ersten Mittel nach Abgabe eines vorgegebenen Befüllungsvölumens an den Filter ein Flüssigkeitsaustritt aus dem zweiten Raum (14) erfasst wird und/oder eine fehlerhafte Befüllung des Filters ermittelt wird, wenn mittels der ersten Mittel während der Abgabe eines vorgegebenen Befüllungsvolumens an den Filter ein Flüssigkeitsaustritt aus dem zweiten Raum (14) erfasst wird.

13. Verfahren zum Befüllen eines medizinischen Filters nach einem der Ansprüche 10 bis 12, **dadurch gekennzeichnet, dass** der Flüssigkeitsaustritt aus wenigstens einem der Räume (12, 14) anhand des Gewichts und/oder Volumens und/oder Durchflusses und/oder Leitfähigkeit der austretenden Flüssigkeit aus dem Filter erfasst wird.

14. Verfahren zum Befüllen eines medizinischen Filters nach einem der Ansprüche 10 bis 13, **dadurch gekennzeichnet, dass** nach dem zweiten Raum (14) eine Auffangvorrichtung zur Aufnahme von aus dem zweiten Raum ablaufender Flüssigkeit vorgesehen ist und dass zur Ermittlung des Flüssigkeitsaustritts das Gewicht der Auffangvorrichtung erfasst und ausgewertet wird.

15. Verfahren zum Befüllen eines medizinischen Filters nach einem der Ansprüche 10 bis 14, **dadurch gekennzeichnet, dass** ein Schwellwert für den Flüssigkeitsaustritt vorgegeben wird und dass ein unterhalb eines vorgegebenen Schwellwertes vorliegender Flüssigkeitsaustritt, der während der Befüllung des Filters (10) auftritt, ausgeblendet wird und für die Beurteilung des Befüllungszustandes unberücksichtigt bleibt.

16. Verfahren zum Befüllen eines medizinischen Filters nach einem der Ansprüche 10 bis 15, **dadurch gekennzeichnet, dass** der Zustand des Ablaufs aus dem zweiten Raum (14) dadurch ermittelt wird, dass ein Teilbefüllungsvolumen für die Befüllung des Filters vorgegeben wird, wobei das Teilbefüllungsvolumen geringfügig größer ist als das Füllvolumen des ersten Raumes (12), und wobei nach Befüllen des ersten Raumes und vor einem Drehen des Filters (10) ein geöffneter Ablauf des zweiten Raumes (14) dadurch erkannt wird, dass nach Abgabe des Teilbefüllungsvolumens ein Flüssigkeitsaustritt aus dem zweiten Raum (14) ermittelt wird und/oder ein geschlossener Ablauf des zweiten Raumes (14) dadurch erkannt wird, dass nach Abgabe des Teilbefüllungsvolumens kein Flüssigkeitsaustritt aus dem zweiten Raum (14) ermittelt wird.

## Claims

1. An apparatus for the filling of a medical filter comprising
at least one filter (10) having at least one first space (12) and one second space (14) which are semipermeably separated by one or more membranes, with the spaces (12, 14) each having at least one inflow and outflow (15, 17, 16, 18); and
wherein the first space (12) can first be filled by means of the apparatus and the filter (10) can be be rotated by approximately 180° after the filling of the first space so that the outflow of the second space is located at the upper end of the dialyzer, and then the second space (14) can be filled by liquid overflow from the first space via the membrane or membrane, and
a detection unit (30) having first and second means, with a liquid discharge from at least the second space (14) being able to be detected by means of the first means and the state of the filing of the filter (10) being able to be determined by means of the second means with reference to the detected liquid discharge, with a filter rotation which has occurred or a missing filter rotation being able to be recognized.

2. An apparatus for the filling of a medical filter in accordance with claim 1, **characterized in that** the inflows and outflows (15, 17, 16, 18) of the first and second spaces are arranged such that the at least one filter (10) is operated in counterflow.

3. An apparatus for the filling of a medical filter in accordance with either of claims 1 or 2, **characterized in that** a filling volume for the filling of the filter (10) is preset and/or stored in the apparatus; and **in that** a correct filling of the filter is determined by means of the second means of the detection unit (30) if a liquid discharge from the second space is detected by means of the first means after dispensing of the filling volume to the filter.

4. An apparatus for the filling of a medical filter in accordance one of the preceding claims, **characterized in that** a filling volume for the filling of the filter (10) is preset and/or stored in the apparatus; and **in that** an incorrect filling of the filter is determined by means of the second means of the detection unit (30) if a liquid discharge from the second space is detected by means of the first means after dispensing of the filling volume to the filter.

5. An apparatus for the filling of a medical filter in accordance with one of the preceding claims, **characterized in that** the first means include at least one sensor which detects the liquid discharge from at least one of the spaces with reference to weight and/or volume and/or flow rate and/or conductivity.

6. An apparatus for the filling of a medical filter in accordance with one of the preceding claims, **characterized in that** a collection apparatus for the reception of liquid flowing out of the second space is provided after the second space; and **in that** the first means include a weighing device (30) or are made as a weighing device which determines the weight of the collection apparatus.

7. An apparatus for the filling of a medical filter in accordance with one of the preceding claims, **characterized in that** the second means are made such that a threshold value for the liquid discharge is stored in the second means and a liquid discharge which is detected by the first means, is below a preset threshold value and occurs during the filling of the filter (10) is discounted and is not taken into account for the evaluation of the filling state.

8. An apparatus for the filling of a medical filter in accordance with one of the preceding claims, **characterized in that** a partial filling volume is preset for the filling of the filter (10), with the partial filling volume being slightly larger than the filling volume of the first space (12), and that the state of the outflow from the second space can be determined by means of the second means of the detection unit, with an open outflow being recognized by the second means if, after dispensing of the partial filling volume, a liquid discharge from the second space is determined by the first means and/or a closed outflow of the second space is recognized if, after dispensing of the partial filling volume, no liquid discharge from the second space is determined by the first means.

9. An apparatus for the filling of a medical filter in accordance with one of the preceding claims, **characterized in that** the apparatus is part of a medical device, in particular of a dialysis machine.

10. A method for the filling of a medical filter (10),
wherein the filter (10) has at least one first space (12) and one second space (14) which are semipermeably separated by one or more membranes (20) and the spaces each have at least one inflow and one outflow (15, 17, 16, 18) and are filled sequentially;
wherein first the first space (12) is filled for filling and the filter (10) is rotated by approximately 180° after the filling of the first space so that the outflow of the second space is located at the upper end of the dialyzer, and then the second space (14) is filled by liquid overflow from the first space via the membrane or membrane, and
wherein the liquid discharge from at least the second space (14) is detected and the state of the filling of the filter is determined with reference to the detected liquid discharge, with a filter rotation which has occurred or a missing filter rotation being recognized.

11. A method for the filling of a medical filter in accordance with claim 10, **characterized in that** the inflows and outflows (15, 17, 16, 18) of the first and second spaces (12, 14) are arranged such that the at least one filter is operated in counterflow.

12. A method for the filling of a medical filter in accordance with claim 10 or claim 11, **characterized in that** a correct filling of the filter (10) is determined if a liquid discharge from the second space (14) is detected by means of the first means after dispensing of a preset filling volume to the filter and/or an incorrect filling of the filter is determined if a liquid discharge from the second space (14) is detected by means of the first means during the dispensing of a preset filling volume to the filter.

13. A method for the filling of a medical filter in accordance with one of the claims 10 to 12, **characterized in that** the liquid discharge from at least one of the spaces (12, 14) is detected with reference to the weight and/or volume and/or flow rate and/or conductivity of the liquid discharged from the filter.

14. A method for the filling of a medical filter in accordance with one of the claims 10 to 13, **characterized in that** a collection device for the reception of liquid flowing out of the second space (14) is provided after the second space; and **in that** the weight of the collection device is detected and evaluated for the determination of the liquid discharge.

15. A method for the filling of a medical filter in accordance with one of the claims 10 to 14, **characterized in that** a threshold value for the liquid discharge is preset; and **in that** a liquid discharge which is below a preset threshold value and occurs during the filling of the filter (10) is discounted and is not taken into account for the evaluation of the filling state.

16. A method for the filling of a medical filter in accordance with one of the claims 10 to 15, **characterized in that** the state of the outflow from the second space (14) is determined **in that** a partial filling volume for the filling of the volume is preset, with the partial filling volume being slightly larger than the filling volume of the first space (12) and with an open outflow of the second space (14) being recognized after the filling of the first space and before a rotation of the filter (10) **in that** a liquid discharge from the second space (14) is determined after dispensing the partial filling volume and/or a closed outflow of the second space (14) is detected **in that** no liquid discharge from the second space (14) is determined after dispensing of the partial filling volume.

## Revendications

1. Dispositif pour remplir un filtre médical, comprenant au moins un filtre (10) doté d'au moins un premier (12) et un second (14) espace, qui sont séparés de manière semi-perméable par une ou plusieurs membranes, les espaces (12, 14) comportant chacun au moins une entrée et une sortie (15, 17, 16, 18), dans lequel, au moyen du dispositif, le premier espace (12) peut être rempli d'abord et après le remplissage du premier espace, le filtre (10) peut être tourné d'environ 180° de telle manière que la sortie du second espace se trouve à l'extrémité supérieure du dialyseur, et ensuite le second espace (14) peut être rempli par le biais de la membrane ou des membranes par le passage du liquide provenant du premier espace,
et
une unité de détection (30) comprenant des premiers et seconds moyens, dans lequel, à l'aide des premiers moyens, une sortie du liquide au moins hors du second espace (14) peut être détectée, et à l'aide des seconds moyens, sur la base de la sortie du liquide détectée, l'état de remplissage du filtre (10) peut être déterminé, une rotation du filtre effectuée ou absente pouvant être détectée.

2. Dispositif pour remplir un filtre médical selon la revendication 1, **caractérisé en ce que** les entrées et sorties (15, 17, 16, 18) du premier et du second espace sont disposées de telle manière que l'au moins un filtre (10) peut fonctionner à contre-courant.

3. Dispositif pour remplir un filtre médical selon la revendication 1 ou 2, **caractérisé en ce qu'**un volume de remplissage pour le remplissage du filtre (10) est prédéfini et/ou est enregistré dans le dispositif et **en ce qu'**un remplissage correct du filtre est déterminé à l'aide des seconds moyens de l'unité de détection (30) quand une sortie du liquide hors du second espace est détectée à l'aide des premiers moyens après la transmission du volume de remplissage au filtre.

4. Dispositif pour remplir un filtre médical selon l'une des revendications précédentes, **caractérisé en ce qu'**un volume de remplissage pour le remplissage du filtre (10) est prédéfini et/ou est enregistré dans le dispositif et **en ce qu'**un remplissage incorrect du filtre est déterminé à l'aide des seconds moyens de l'unité de détection (30) quand une sortie du liquide hors du second espace est détectée à l'aide des premiers moyens pendant la transmission du volume de remplissage au filtre.

5. Dispositif de remplissage d'un filtre médical selon l'une des revendications précédentes, **caractérisé en ce que** les premiers moyens comprennent au moins un capteur, qui détecte la sortie du liquide hors d'au moins un des espaces sur la base du poids et/ou du volume et/ou du débit et/ou de la conductivité.

6. Dispositif pour remplir un filtre médical selon l'une des revendications précédentes, **caractérisé en ce qu'**après le second espace, un dispositif de réception est prévu pour recevoir du liquide s'écoulant du second espace et **en ce que** les premiers moyens comprennent un dispositif de pesage (30) ou sont réalisés sous la forme d'un dispositif de pesage, qui détermine le poids du dispositif de réception.

7. Dispositif pour remplir un filtre médical selon l'une des revendications précédentes, **caractérisé en ce que** les seconds moyens sont réalisés de telle manière qu'une valeur seuil pour la sortie du liquide est enregistrée dans les seconds moyens et qu'une sortie du liquide détectée par les premiers moyens située sous une valeur seuil prédéfinie qui se produit pendant le remplissage du filtre (10) est occultée et n'est pas prise en compte pour l'évaluation de l'état de remplissage.

8. Dispositif pour remplir un filtre médical selon l'une des revendications précédentes, **caractérisé en ce qu'**un volume de remplissage partiel est prédéfini pour le remplissage du filtre (10), le volume de remplissage partiel étant légèrement supérieur au volume de remplissage du premier espace (12), et l'état de la sortie du second espace pouvant être déterminé à l'aide des seconds moyens, une sortie ouverte étant détectée par les seconds moyens quand une sortie du liquide hors du second espace est déterminée par les premiers moyens après la transmission du volume de remplissage partiel et/ou une sortie fermée du second espace étant détectée quand aucune sortie du liquide hors du second espace n'est déterminée par les premiers moyens après la transmission du volume de remplissage partiel.

9. Dispositif pour remplir un filtre médical selon l'une des revendications précédentes, **caractérisé en ce que** le dispositif fait partie d'un appareil médical, en particulier d'une machine de dialyse.

10. Procédé pour remplir un filtre médical (10), le filtre (10) comportant au moins un premier (12) et un second (14) espace, qui sont séparés de manière semi-perméable par une ou plusieurs membranes (20), et les espaces comportant chacun au moins une entrée et une sortie (15, 17, 16, 18) et étant remplis l'un après l'autre,
dans lequel, pour le remplissage, le premier espace (12) est d'abord rempli et après le remplissage du premier espace, le filtre (10) est tourné d'environ 180° de telle manière que la sortie du second espace se trouve à l'extrémité supérieure du dialyseur, et ensuite le second espace (14) est rempli par le biais de la membrane ou des membranes par le passage du liquide provenant du premier espace,
et
dans lequel la sortie du liquide au moins hors du second espace (14) est détectée et l'état de remplissage du filtre est déterminé sur la base de la sortie de liquide détectée, rotation du filtre effectuée ou absente étant détectée.

11. Procédé pour remplir un filtre médical selon la revendication 10, **caractérisé en ce que** les entrées et sorties (15, 17, 16, 18) du premier et du second espace (12, 14) sont disposées de telle manière que l'au moins un filtre fonctionne à contre-courant.

12. Procédé pour remplir un filtre médical selon la revendication 10 ou 11, **caractérisé en ce qu'**un remplissage correct du filtre (10) est déterminé quand une sortie du liquide hors du second espace (14) est détectée à l'aide des premiers moyens après la transmission d'un volume de remplissage prédéfini au filtre, et/ou un remplissage incorrect du filtre est déterminé quand une sortie du liquide hors du second espace (14) est détectée à l'aide des premiers moyens pendant la transmission d'un volume de remplissage prédéfini au filtre.

13. Procédé pour remplir un filtre médical selon l'une de revendication 10 à 12, **caractérisé en ce que** la sortie du liquide hors d'au moins un des espaces (12, 14) est détectée sur la base du poids et/ou du volume et/ou du débit et/ou de la conductivité du liquide sortant du filtre.

14. Procédé pour remplir un filtre médical selon l'une des revendications 10 à 13, **caractérisé en ce qu'**un dispositif de réception est prévu après le second espace (14) pour recevoir du liquide s'écoulant du second espace et **en ce que** le poids du dispositif de réception est détecté et évalué pour déterminer la sortie du liquide.

15. Dispositif pour remplir un filtre médical selon l'une des revendications 10 à 14, **caractérisé en ce qu'**une valeur seuil est prédéfinie pour la sortie du liquide et **en ce qu'**une sortie du liquide se situant sous une valeur seuil prédéfinie, qui se produit pendant le remplissage du filtre (10), est occultée et n'est pas prise en compte pour l'évaluation de l'état de remplissage.

16. Procédé de remplissage d'un filtre médical selon l'une des revendications 10 à 15, **caractérisé en ce que** l'état de la sortie du second espace (14) est déterminé par le fait qu'un volume de remplissage partiel est prédéfini pour le remplissage du filtre, le volume de remplissage partiel étant légèrement supérieur au volume de remplissage du premier espace (12), et après le remplissage du premier espace et avant une rotation du filtre (10), une sortie ouverte du second espace (14) est détectée par le fait qu' une sortie du liquide hors du second espace (14) est déterminée après la transmission du volume de remplissage partiel et/ou une sortie fermée du second espace (14) est détectée par le fait qu'aucune sortie de liquide hors du second espace (14) n'est déterminée après la transmission du volume de remplissage partiel.
